# EUROPEAN PATENT APPLICATION

(11) **EP 2 047 809 A1**
(43) Date of publication of application: **15.04.2009**
(21) Application number: 07721737.0
(22) Date of filing: 14.06.2007
(51) Int. Cl.: A61B 17/34, A61M 39/22, A61B 17/94

(54) **ONE-WAY VALVE FOR TROCAR**

(30) Priority: 31.07.2006 CN 200610036835
(71) Applicant: Zhou, Taili, Guangdong 510060 (CN); Li, Yanfang, Guangdong 510060 (CN)
(72) Inventor: Zhou, Taili, Guangdong 510060 (CN); Li, Yanfang, Guangdong 510060 (CN)
(74) Representative: Papa, Elisabetta
(86) International application number: PCT/CN2007/070116
(87) International publication number: WO 2008/017254

(57) **Abstract**

An one-way valve for trocar is provided, which is mounted in the cavity of the end portion of the trocar sheath tube (2), and includes a V-shaped funnel construction (11) acted as the upper portion of the one-way valve (1), a slit (12) provided on the bottom surface of the V-shaped funnel construction (11), and a supporting body (13) acted as the lower portion of the one-way valve (1) and providing the sealing pressure to the slit (12). The supporting body (13) is connected to the outside of the inclined plane of the V-shaped funnel construction (11). Because the supporting body (13) which can provide the sealing pressure to the slit (12) is provided, when the one-way valve (1) is longitudinally compressed, the unique design of the supporting body (13) will provide sealing pressure on the slit (12). This is due to the spring back force formed when the valve is compressed.

## Description

### TECHNICAL FIELD

This invention relates to a medical device, specifically a trocar, which is used in minimally invasive surgeries such as Laparoscopy, Thoracoscopy and so on.

### BACKGROUND ART

Minimally invasive surgeries such as Laparoscopy and Thoracoscopy are now a common practice in modern medicine. To reduce iatrogenic infection; single-use trocars are now in wide use. This patent targets improvements to the trocar by making the structure simple, reducing the cost, while ensuring good performance during the procedure.

### Technical Problem

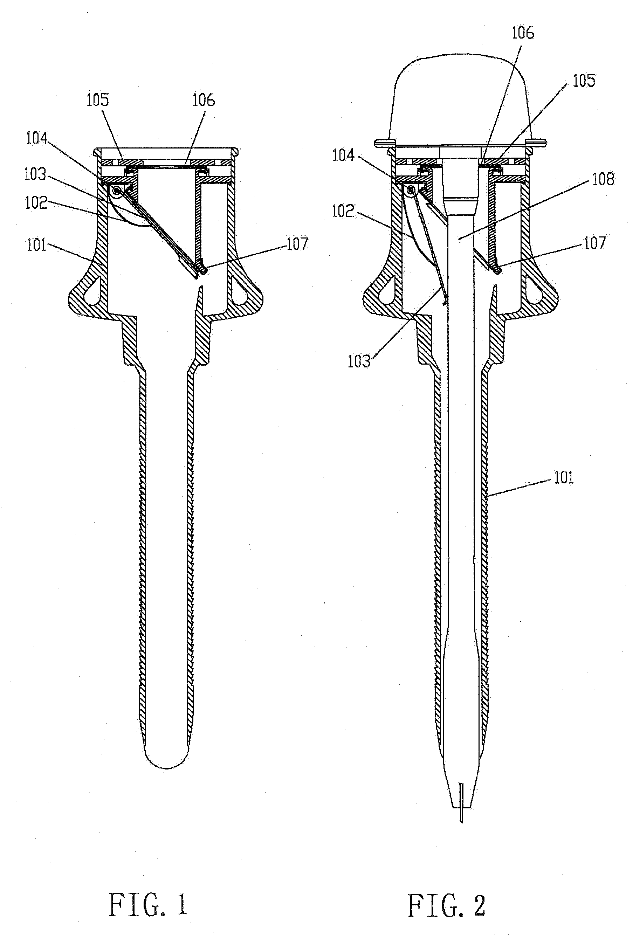

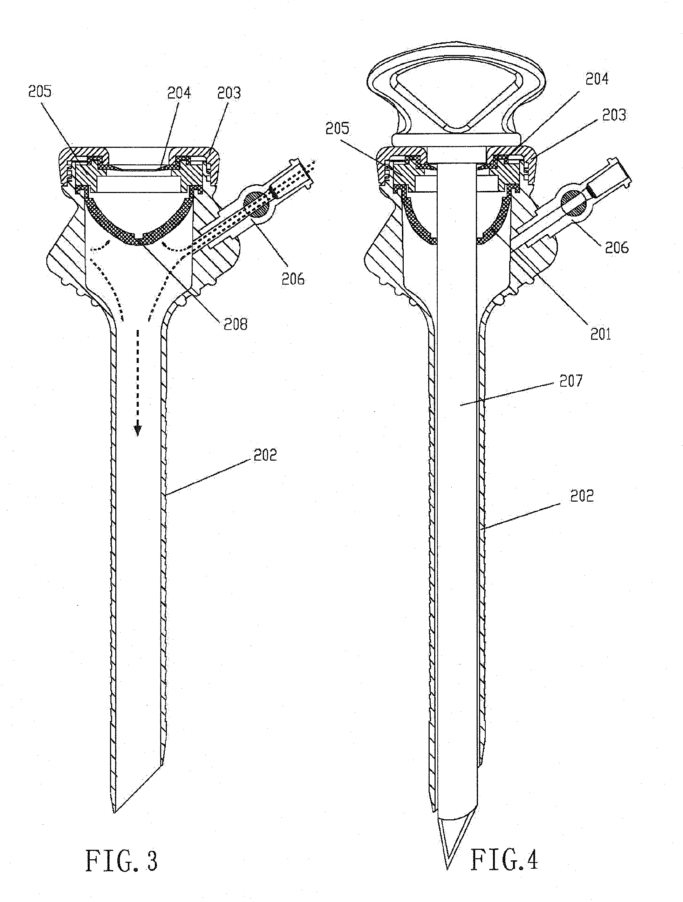

There are two kinds of one-way valves in the art. One is a spring pressed leaf structure (Fig. 1 and Fig. 2). The spring leaf design helps force the baffle-plate to press against the silica gel seal ring; thereby producing a better seal. The complete sealing ring mechanism is comprised of the spring leaf structure, baffle-plate, and the silica gel seal ring. The spring leaf structure and baffle-plate are made of stainless steel. This type of seal ring is commonly used in the reusable trocars made out of metal. These metal trocars are typically heavy and expensive. The recent trend to develop single-use trocars has resulted in a new hourglass shaped silica gel seal ring (Fig. 3 and Fig. 4) to be developed. This hourglass shaped silica gel seal ring has a linear incision penetrating the seal. The design utilizes the contractility of the silica gel along with the pressure formed by the CO₂ pneumoperitoneum to make the seal. This hourglass shaped silica gel seal ring concept is now widely used in single-use trocars. However, the resilience of the silica gel and the sealing pressure formed by the CO₂ pneumoperitoneum are not sufficient, therefore the sealing effect is often inadequate and prone to leakage.

### SUMMARY OF THE INVENTION

### Technology Solution

The purpose of the present invention is to provide a new one-way valve to address some improvements over the prior art. The new one-way valve is simple, reliable, low cost, and has good sealing properties.

In the present invention, a new one-way valve for the trocar is provided. This valve is mounted in the cavity located at the proximal end of the trocar sheath tube. The valve comprises: V-shaped funnel construction acting as the upper portion of said one-way valve; a slit cut into the bottom surface of the V-shaped funnel construction; and a supporting body acting as the lower portion of the one-way valve and providing the additional sealing pressure to the slit. The supporting body is connected to the outside of the inclined plane of the V-shaped funnel construction.

The height of said one-way valve is of equal or higher height to the cavity at the proximal end of the trocar sheath tube for mounting the one-way valve.

The said supporting body includes but is not limited to the following structures:

The said supporting body is comprised of a Λ-shaped structure formed by supporting planes or supporting arms or supporting rods matched to each other. The upper portion and lower portion of the one-way valve are connected into an X-shaped funnel. Due to this design, when the one-way valve is compressed along the longitudinal axis, there is additional pressure applied to the slit, resulting in an improved sealing effect.

The said one-way valve also includes a hole in the supporting body, which allows gas to pass through. The hole can be made in the supporting planes, supporting arms, or supporting rods. The hole can also be formed in the intervening space between the supporting planes, supporting arms, or supporting rods.

The said one-way valve is further comprised of a supporting tendon on the inner side of said supporting body.

The said one-way valve is further comprised of a secondary tendon located on the outer portion of the supporting body.

The supporting tendon and secondary tendon act to further enhance the sealing pressure on the slit. When the one-way valve according to the present invention is compressed and mounted in the cavity of the end of the sheath tube of trocar, because the diameter of the position where the assistant tendon is mounted is larger than the diameter of the cavity of the end of the sheath tube of trocar, the pair of the assistant tendons, which are matched each other, of the supporting body can engender longitudinal pressure. This longitudinal pressure can be passed to the inclined plane of V-shaped funnel construction through the supporting tendon of the supporting body, and provide sealing pressure to the slit.

The said supporting body is a tube-shaped structure with a circular or elliptical cross section. When the one-way valve is longitudinally compressed, the one-way valve providessealing pressure to the slit, thereby achieving the sealing effect. This sealing pressure is an inherent result of the resilience created when the circular or elliptical tube structure is deformed. In addition, the V-shaped funnel construction with supporting tendons of the supporting body provides further resilience once longitudinally compressed and deformed, thereby providing additional sealing pressure to the slit.

The one-way valve according to the present invention further comprises a hole through which gases are allowed to pass through. This hole is located on the supporting body.

The one-way valve according to the present invention further comprises a reinforcing tendon, which is provided on the V-shaped wall of V-shaped funnel construction. The reinforcing tendon is used to improve the rigidity of the V-shaped wall.

The one-way valve according to the present invention is made of flexible medical materials selected from a group of medical silica gel, medical Polyurethane, and medical rubber.

During assembly of the trocar, the said one-way valve is mounted in the cavity of the proximal end of the trocar sheath. Since the designed height of the valve is of equal height or higher than the cavity, once mounted and cap of the trocar placed, the valve is longitudinally compressed. This creates pressure on the valve resulting in greater resilience and improved sealing pressure of the slit in the one-way valve. Namely, the said one-way valve is longitudinally compressed while being mounted into the proximal portion of the trocar sheath.

After the puncturing pore is inserted into the trocar sheath, the slit is split open, allowing the puncturing pore to pass. Meanwhile the ring seal on the upper cover encloses around the puncturing pore helping to prevent gas leakage in the trocar. Once the puncturing pore is pulled out, the ring seal on the upper cover cannot produce a sealing effect, only the one-way valve can do this. This is due to the longitudinal compression of the valve in the trocar cavity providing sealing pressure to the slit. In addition, the gas pressure of the CO₂ pneumoperitoneum on the walls of the V-shaped funnel structure of the one-way valve also provides sealing pressure to the slit. The pressures achieved in the pneumoperitoneum are too low to provide adequate sealing force by itself.

The one-way valve of the trocar described in this invention is simple, reliable, low cost, and provides a good sealing effect. This valve is able to sustain good sealing pressures even after 100,000 passages of surgical instruments. The one-way valve as described in the present invention can be widely used in many types of Minimally Invasive Surgery; such as Laparoscopy, Thorascopy and so forth.

### Brief description of the Drawings

Fig.1 is a perspective view illustrating structure of trocar with a one-way valve made from spring leaf, baffle and silica gel seal ring structure in the art.
Fig.2 is a perspective view illustrating structure of state of the trocar with a one-way valve made from spring leaf, baffle and silica gel seal ring structure in the art after inserting a puncturing pole.
Fig.3 is a perspective view illustrating structure of trocar which has applied hourglass shape silica gel one-way valve in the art.
Fig.4 is perspective view illustrating structure of trocar which has applied hourglass shape silica gel one-way valve in the art, after inserting a puncturing pole.
Fig.5 is a perspective view illustrating structure of hourglass shape silica gel one-way valve in the art.
Fig.6 is A-A cutaway view of Fig.5.
Fig.7 is B-B cutaway view of Fig.5.
Fig.8 is a perspective view illustrating structure of the said X-shaped one-way valve according to the present invention.
Fig.9 is front view of Fig.8.
Fig.10 is Right View of Fig.9.
Fig.11 is vertical view of Fig. 10.
Fig.12 is C-C cutaway view of Fig. 10.
Fig.13 is the cube display view top of Fig.8.
Fig.14 is the cube display view underside portion of Fig.8.
Fig.15 is the bottom view of Fig.8.
Fig.16 is a perspective view illustrating structure of the one-way valve with a Λ-shaped supporting body according to the present invention.
Fig.17 is the cube display view of Fig.16.
Fig.18 is front view of Fig.16.
Fig.19 is D-D cutaway view of Fig.17.
Fig.20 is a perspective view illustrating structure of the one-way valve according to the present invention, with rod-like Λ-shaped supporting body according to the present invention.
Fig.21 is the cube display view of Fig.20.
Fig.22 is the cube display view underside portion of Fig.20.
Fig.23 is E-E cutaway view of Fig.21.
Fig.24 is a perspective view illustrating structure of the one-way valve with a tube-shaped supporting body with a circular or elliptical cross section according to the present invention.
Fig.25 is the cube display view of Fig.24.
Fig.26 is front view of Fig.24.
Fig.27 is F-F cutaway view of Fig.25.
Fig.28 is a perspective view illustrating structure of the improved product of X-shaped one-way valve according to the present invention.

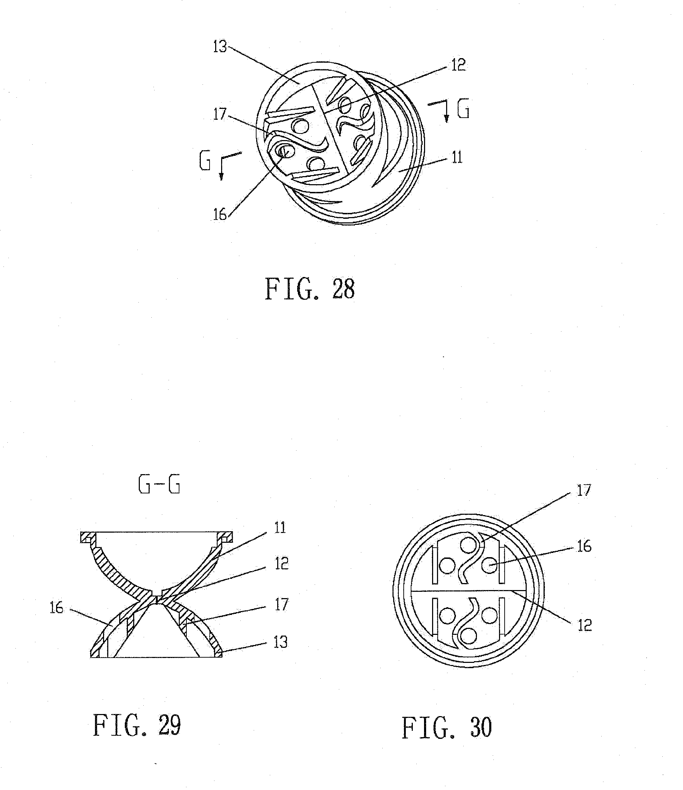

Fig.29 G-G cutaway view of Fig.28
Fig.30 is the bottom view of Fig. 28.

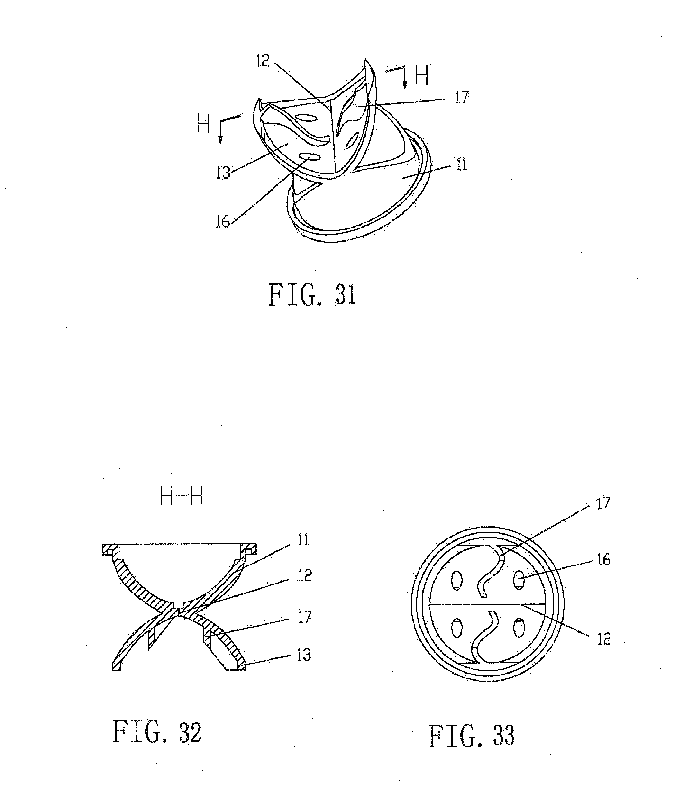

Fig.31 is a perspective view illustrating structure of the improved product of one-way valve with a Λ-shaped supporting body according to the present invention.
Fig.32 is H-H cutaway view of Fig.31.
Fig.33 is the bottom view of Fig.31.

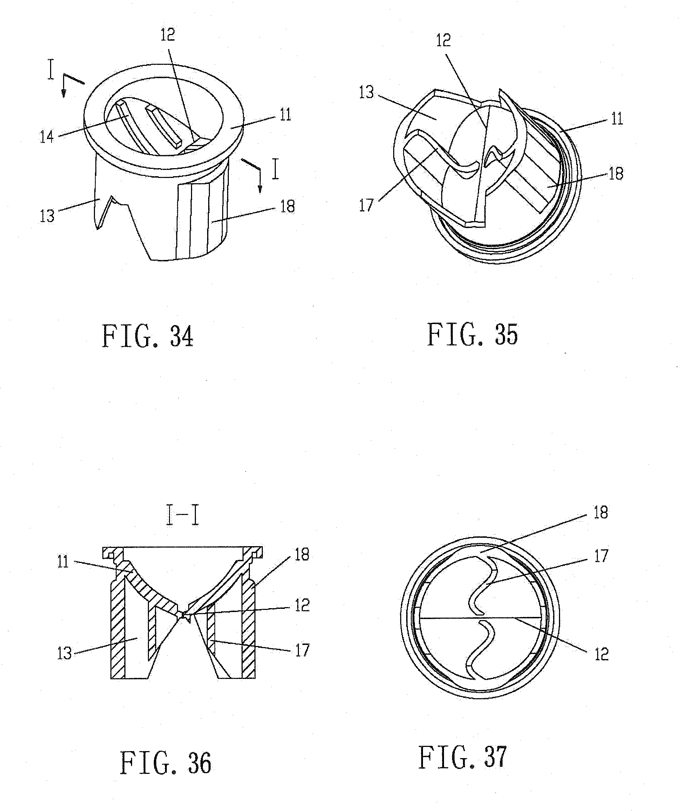

Fig.34 is a perspective view illustrating structure of the compound one-way valve with a radially thickened supporting body according to the present invention.
Fig.35 is the cube display view of Fig.34, the bottom of the one-way valve is upward in the Fig.34.
Fig.36 is I-I cutaway view of Fig.34.
Fig.37 is the bottom view of Fig.34.

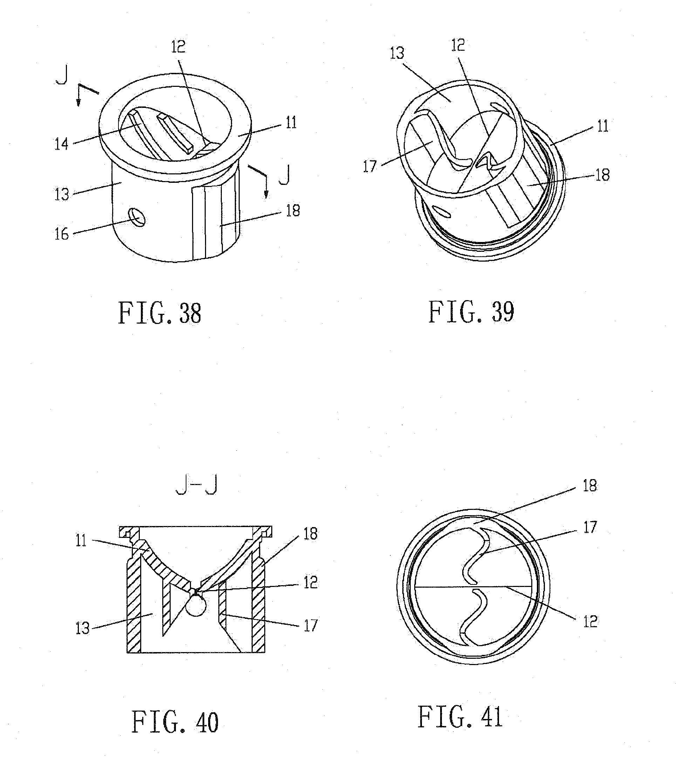

Fig.38 is a perspective view illustrating structure of the compound one-way valve with a radially thickened supporting body with holes.
Fig.39 is the cube display view of Fig.38, the bottom of the one-way valve is upward in the Fig.39.
Fig.40 is the J-J cutaway view of Fig.38.
Fig.41 is the bottom view of Fig.38.

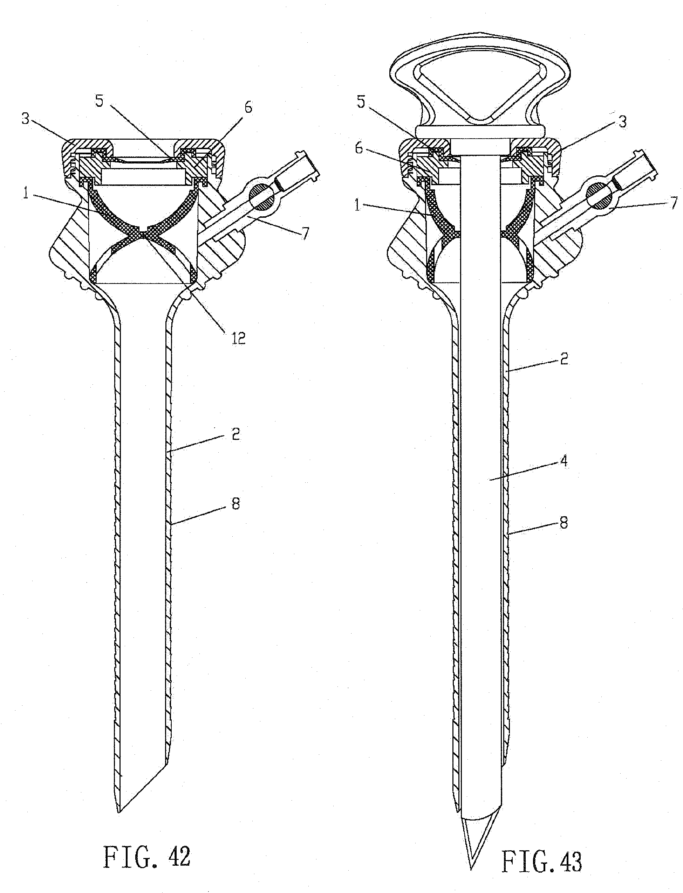

Fig.42 is a perspective view illustrating structure of trocar mounted the said one-way valve according to the present invention.
Fig.43 is a perspective view illustrating structure of the state of trocar said in Fig.42 according to the present invention after inserting a puncturing pole.

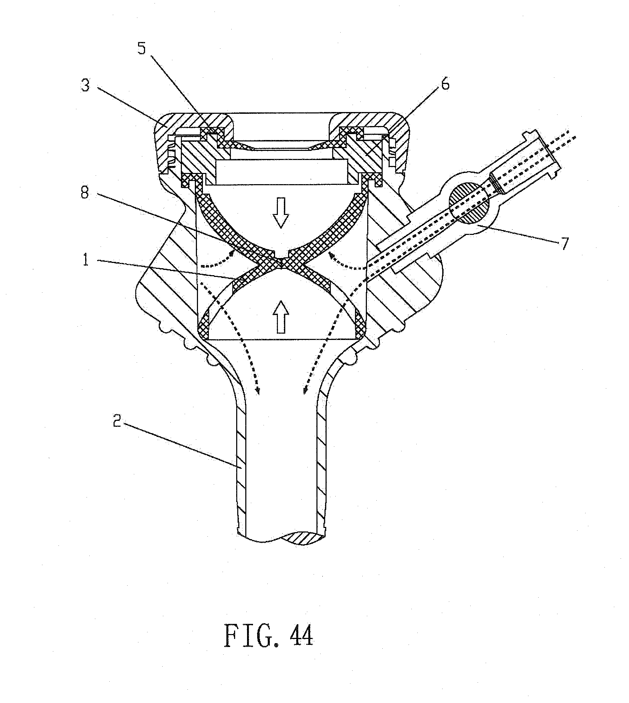

Fig.44 is a perspective view illustrating the seal principle of trocar mounted the said one-way valve according to the present invention.

In the above figures,
1 denotes the one-way valve according to the present invention; 2 denotes the sheath tube of trocar; 3 denotes the upper cover; 4 denotes the puncturing pole; 5 denotes the seal ring of upper cover; 6 denotes locating block; 7 denotes ventilated valve; 8 denotes antiskid strips; 11 denotes V-shaped funnel construction; 12 denotes slit; 13 denotes supporting body; 14 denotes enhancing tendon; 151 denotes supporting plane; 152 denotes supporting arm; 153 denotes supporting rod; 16 denotes accessing hole; 17 denotes supporting tendon; 18 denotes secondary tendon. The arrowhead of broken line denotes the flowing direction of CO₂ gas, and the arrowhead of real line denotes the longitudinal pressed direction of the one-way valve.

101 denotes the sheath tube of trocar in the art; 102 denotes the upper cover of trocar in the art; 103 denotes spring leaf of trocar in the art; 104 denotes seal baffle of trocar in the art; 105 denotes seal ring on the seal baffle of trocar in the art; 106 denotes seal ring on the upper cover of trocar in the art; 107 denotes locating pin on the spring leaf of trocar in the art; 108 denotes puncturing pole of trocar in the art.

201 denotes the hourglass-shaped one-way valve of single-use trocar in the art; 202 denotes the sheath tube of single-use trocar in the art; 203 denotes the upper cover of single-use trocar in the art; 204 denotes the seal ring on the upper cover of single-use trocar in the art; 205 denotes locating block of single-use trocar in the art; 206 denotes ventilated valve of single-use trocar in the art; 207 denotes puncturing pole of single-use trocar in the art; 208 denotes a slit on the hourglass-shaped one-way valve of single-use trocar in the art; 209 denotes enhancing tendon on the on the hourglass-shaped one-way valve of single-use trocar in the art.

### Best Mode for Carrying Out the Invention

### Example 1: X-shaped one-way valve

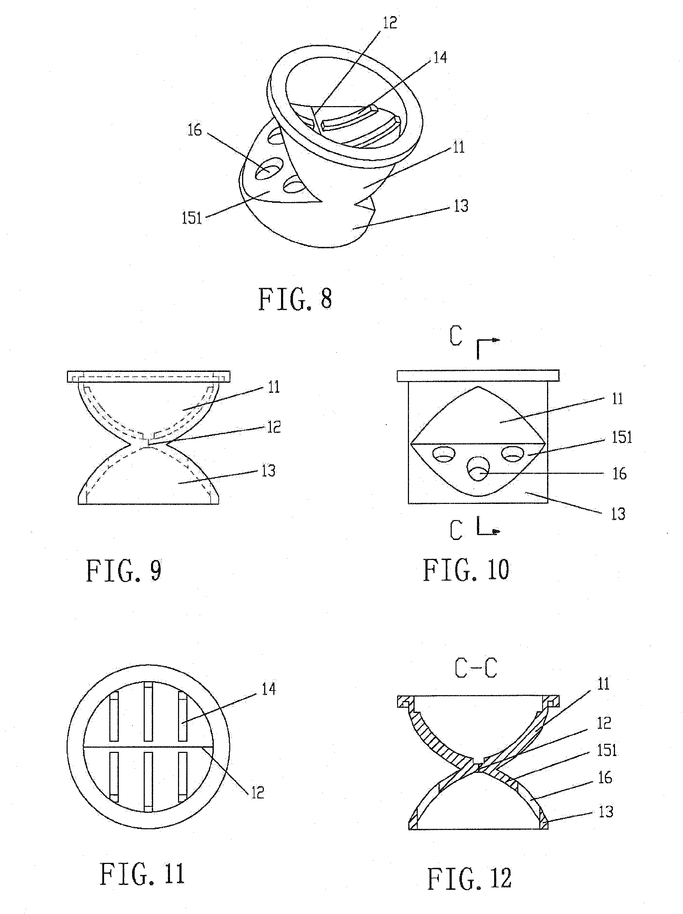

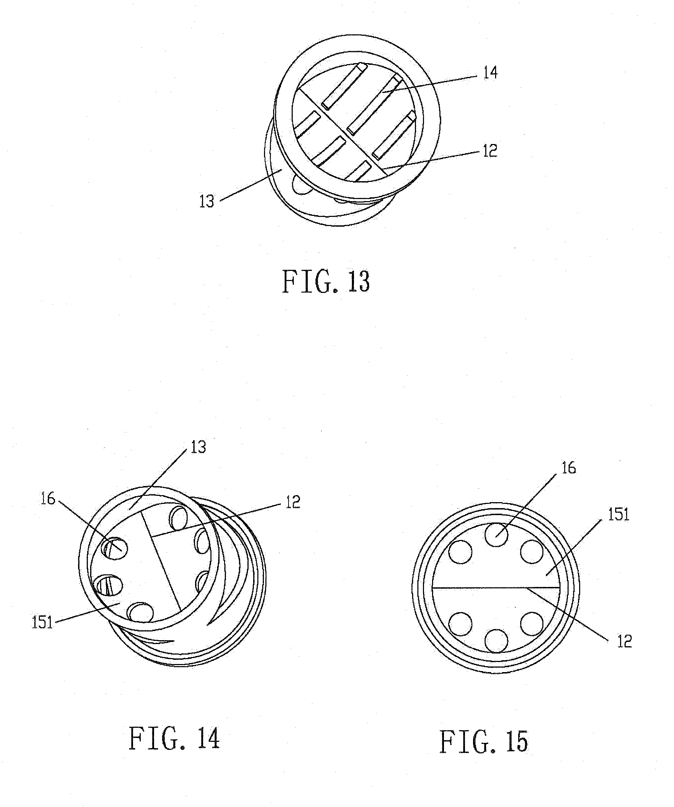

The product shown in Fig.8 is the basic product type designed according to the present invention, and it is called X-shaped one-way valve. The upper portion of this one-way valve is a V-shaped funnel construction 11. There is a perforative slit, which is usually a linear shape one, on the bottom of V-shaped funnel construction 11, and an enhanced tendon 14 is mounted on the wall of V-shaped funnel construction 11. The underside portion of one-way valve is a Λ-shaped structure formed by supporting plane 151 which matches each other, and which is similar to an inverted funnel, acting as supporting body 13. The Upper portion which is V-shaped funnel construction 11 and the underside portion which is Λ-shaped supporting body 13 form the one-way valve 1 with an X-shaped cross-section. The accessing hole 16 is mounted on the supporting plane 151 for the flowing of the CO₂ gas. See Fig.8 to Fig.15. After the mold is prepared according to the above product design, the X-shaped one-way valve said in the present invention can be manufactured according to the standard manufacture method of medical silica gel products.

### Example 2: The one-way valve with a Λ-shaped supporting body

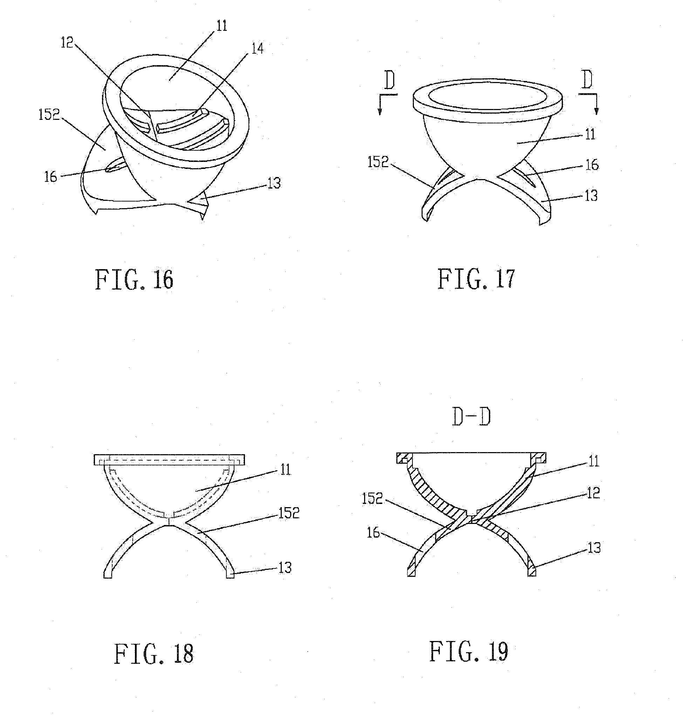

The product shown in Fig.16 is another product type designed according to the present invention, and it is called one-way valve with Λ-shaped supporting body. The upper portion of this type product is the same as that of X-shaped one-way valve in Example 1, but the underside portion is different. In the underside portion, the Λ-shaped structure is formed by two supporting arms 152 matching each other, which is saddle-like or arch-like. The Λ-shaped structure acts as supporting body 13. The upper portion of V-shaped funnel construction 11 and the underside portion of the saddle-like Λ-shaped structure form the said one-way valve with Λ-shaped supporting body. The specific manufacture method is the same as that of the Example 1. See Fig.16 to Fig.19.

### Example 3: The one-way valve with a rod-like Λ-shaped supporting body

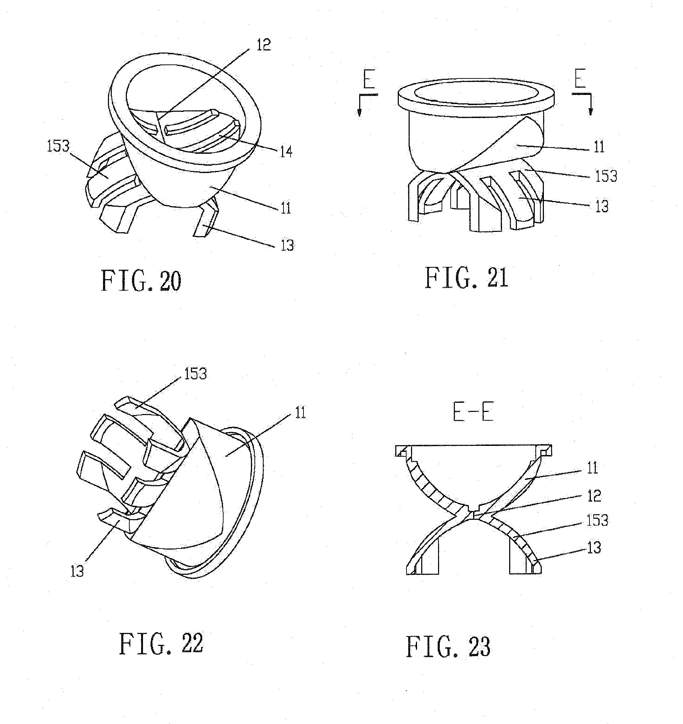

The one-way valve shown in Fig.20 is the third product type designed according to the present invention, and it is called one-way valve with rod-like Λ-shaped supporting body. The upper portion of this type of one-way valve is the same as that of X-shaped one-way valve in Example 1, but the underside portion is different. In the underside portion, the Λ-shaped structure is formed by multiple supporting rods 153 which match each other. This structure acts as supporting body. The upper portion of V-shaped funnel construction 11 and the underside portion of the Λ-shaped structure with multiple supporting rods 153 form the one-way valve 1 with rod-like Λ-shaped supporting body. The specific manufacture method is the same as the Example 1. See Fig.20 to Fig.23.

### Example 4: The one-way valve with a tube-shaped supporting body with a circular or elliptical cross-section

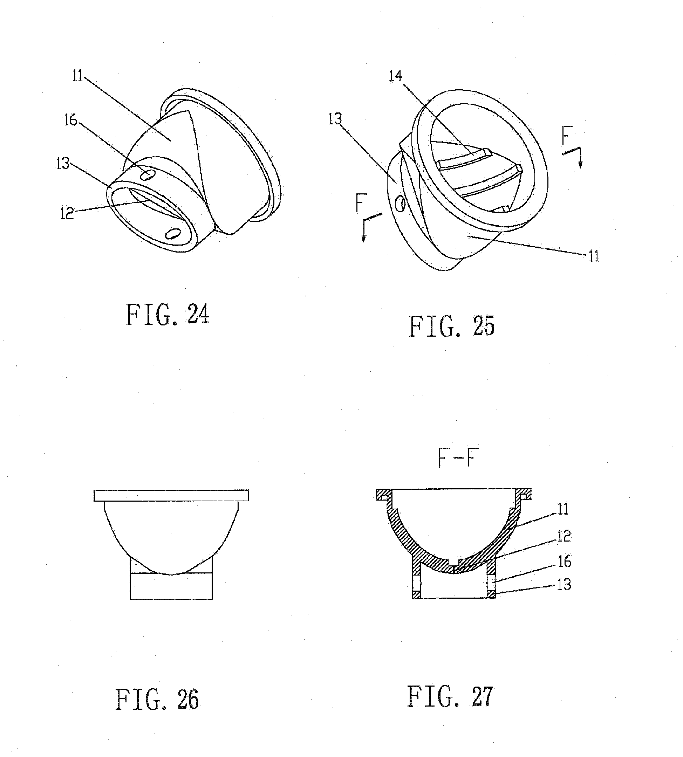

The product shown in Fig.24 is the fourth product type designed according to the present invention, and it is called one-way valve with a tube-shaped supporting body with a circular or elliptical cross-section. The upper portion of this type product is the same as that of X-shaped one-way valve in Example 1, but the underside portion is different. In this product type, the underside portion is a tube-shaped structure with a circular or elliptical cross-section, forming the supporting body. The upper portion of the V-shaped funnel construction 11 and the underside portion of a supporting body 13 of the tube-shaped structure with a circular or elliptical cross-section form the one-way valve with a tube-shaped supporting body with a circular or elliptical cross-section. The specific manufacture method is the same as the Example 1. See Fig.24 to Fig.27.

### Example 5: Improved product of X-shaped one-way valve

Fig.28 shows improved product of X-shaped one-way valve based on the Example 1. The improvements of this one-way valve 1 are as follow: Supporting tendon 17 is mounted on the underside of the supporting body 13. The two S-shaped supporting tendons shown in Fig.28 are connected to the outer of the said V-shaped funnel construction 11, and mounted on the two sides of the linear slit 12 respectively. The said supporting tendon 17 can enhance the sealing pressure to slit 12. With the additional structure of supporting tendon 17, the position of the accessing hole 16 in the supporting body 13 is adjusted accordingly. See Fig.28 to Fig.30. The other structure of this one-way valve and its manufacture method are the same as those the Example 1.

### Example 6: The improved product of one-way valve with Λ-shaped supporting body

Fig.31 shows the improved product of one-way valve with Λ-shaped supporting body based on the Example 2. The improvements of this one-way valve are as follow: Supporting tendon 17 is mounted on the underside of the supporting body 13. The two S-shaped supporting tendons shown in Fig.31 are connected to the outer of the said V-shaped funnel construction 11, and mounted on the two sides of the linear slit 12 respectively. The said supporting tendon 17 can enhance the sealing pressure to slit 12. With the additional structure of supporting tendon 17, the position of the accessing hole 16 in supporting body 13is adjusted accordingly. See Fig.31 to Fig.33. The other structure of this one-way valve and its manufacture method are the same as those in the Example 2.

### Example 7: Compound one-way valve with a radially thickened supporting body

Fig.34 shows the compound one-way valve with a radially thickened supporting body according to the present invention. The V-shaped funnel construction 11 of this product type is the same as that in Example 1, while the structure of the supporting body 13 is different. The supporting body 13 in this example comprises two wider supporting planes which match each other and the supporting tendon 17. The supporting tendon 17 connects the inner part of supporting plane and outer part of the inclined plane of V-shaped funnel construction 11. The secondary tendon 18 which is of certain thickness is mounted on the outer part of supporting plane, vertical to slit 12. When this example product type is compressed in the cavity of the end portion of the trocar sheath tube, as the diameter of the secondary tendon 18 is bigger than that of the cavity of the end portion of the trocar sheath tube, this pair of secondary tendon 18 which match each other will create radial pressure. This radial pressure can be transferred to the inclined plane of V-shaped funnel construction 11 through supporting tendon 17, and thus forms a sealing pressure to the slit 12. See Fig.34 to Fig.37.

The other structure of this one-way valve and its manufacture method is the same as those in the Example 1.

### Example 8: Compound one-way valve with a radially thickened supporting body with holes

Fig.38 shows the compound one-way valve with a radially thickened supporting body with holes according to the present invention. The V-shaped funnel construction 11 of this product type is the same as that in Example 1, while the structure of the supporting body 13 is different.

The supporting body 13 in this example comprises cylindrical supporting plane and supporting tendon 17. Supporting tendon 17 connects the inner part of supporting plane and outer part of the inclined plane of V-shaped funnel construction 11. The secondary tendon 18 which is of certain thickness is mounted on the outer part of the supporting plane, vertical to slit 12. There are accessing holes 16 on the supporting plane for transporting CO₂.

When this example product type is compressed in the cavity of the end portion of the trocar sheath tube 2, as the diameter of the secondary tendon 18 is bigger than that of the cavity of the end portion of the trocar sheath tube, this pair of secondary tendon 18 which match each other will create radial pressure. This radial pressure can be transferred to the inclined plane of V-shaped funnel construction 11 through supporting tendon 17, and thus forms sealing pressure to the slit 12. See Fig.38 and Fig.41. The other structure of this one-way valve and its manufacture method are the same as those in the Example 1.

### Example 9: Trocar with the one-way valve in the present invention installed.

According to the technical scheme of the present invention, the said one-way valve 1 should be manufactured by using soft medical materials, such as medical-grade silica gel, medical Polyurethane, medical rubber. According to the design scheme of current technology, choose medical plastic to manufacture the sheath tube 2, upper cover 3, puncturing pore 4, upper cover seal ring 5, locating block 6, ventilated valve 7 of the trocar, and assemble them according to the drawing. Firstly, mount the said one-way valve 1 in the cavity of the end portion of the trocar sheath tube 2. The height of the one-way valve 1 is a little more than the size of cavity which is used for mounting one-way valve 1 in the end portion of the trocar sheath tube 2; and mounts the locating block by latch match method.

The locating block can also be mounted on the end portion of the trocar sheath tube 2 by ultrasonic welding, to make the one-way valve which is mounted in the cavity of the end portion of the trocar sheath tube 2 be longitudinally compressed. In this way, sealing pressure is provided to the slit 12 of the one-way valve 1 by the resilience of the medical silica gel to form a supporting body. Finally, mount the upper cover seal ring 5, upper cover 3 and ventilated valve 7. The upper cover 3 can be connected to the end portion of the trocar sheath tube by screw thread, or ultrasonic welding, or adhesive; ventilated valve 7 should be mounted on the reserved place of the end portion of the trocar sheath tube 2 by ultrasonic welding, or adhesive. Ventilated valve 7 can be commonly used 2-way valve or 3-way valve for medicine purposes. See Fig.42 and Fig.44.

For enhancing the antiskid performance of the trocar sheath tube 2, antiskid strips 8 which is ripple-shaped or rough processed can be made on the front part of the trocar sheath tube 2. See Fig.42 and Fig.43.

After the puncturing pore 4 is inserted into the trocar sheath tube 2and the slit 12 is split let the puncturing pore 4 pass. Meanwhile, the seal ring 5 on the upper cover surrounds the puncturing pore 4 to make the trocar sealed and prevent it from gas leakage. See Fig.43.

When the puncturing pore 4 is pulled out, the sealing effect is performed by the one-way valve 1, not by the seal ring 5 on the upper cover. The reason is that when the one-way valve 1 which is mounted in the cavity of the end portion of the trocar sheath tube 2 is longitudinally compressed, the one-way valve 1 can provide sealing pressure to the slit 12 as a result of the resilience force of the medical silica gel, and thus the sealing effect can be achieved.

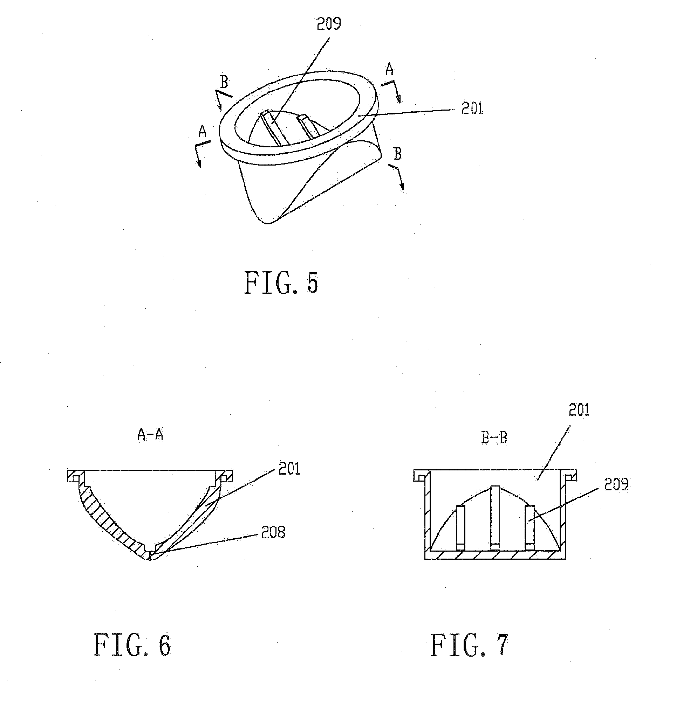

Meanwhile, the gas pressure of the CO₂ pneumoperitoneum onto the wall of V-shaped funnel construction 11 of one-way valve 1 can also provide sealing pressure to the slit 12, as shown in Fig.42 and Fig.44. While in the funnel-shaped one-way valve 201 which is state-of-the-art, only the gas pressure of the CO₂ pneumoperitoneum can provide sealing pressure to the slit 208. As a result of low gas pressure of CO₂ pneumoperitoneum, effective sealing effect usually can not be achieved, as shown in Fig.3 to Fig.7.

The air-tightness of the said one-way valve 1 in the present invention and the trocar which adopts the said one-way valve is better than the funnel-shaped one-way valve 201 which is state-of-the-art. The said one-way valve in the present invention is not only ingenious and reliable, but also has good sealing effect with low cost. The trocar which adopts the said one-way valve in the present invention can still keep a good sealing performance after the surgical device has repeatedly passed in and out 100,000 times. The one-way valve in the present invention can be widely used in trocars used in minimally invasive surgery, such as laparoscopy, thoracoscopy and so on.

While the invention has been particularly shown and described with reference to preferred embodiments thereof, it will be understood by those skilled in the art that the foregoing and other changes in form and details may be made therein without departing from the spirit and scope of the invention.

## Claims

1. An one-way valve for trocar, mounted in the cavity located at the proximal end of the trocar sheath tube (2), the valve comprising:
a V-shaped funnel construction (11), acted as the upper portion of said one-way valve (1);
a slit (12), cut into the bottom surface of said V-shaped funnel construction (11); and
a supporting body (13), acted as the lower portion of the one-way valve (1) and providing the sealing pressure to the slit (12);
wherein said supporting body (13) is connected to the outside of the inclined plane of said V-shaped funnel construction (11).

2. The one-way valve according to claim 1, wherein the height of said one-way valve (1) is of equal or higher height to the cavity at the proximal end of the trocar sheath tube (2) for mounting the one-way valve(1).

3. The one-way valve according to claim 1, wherein said supporting body (13) comprising a Λ-shaped structure formed by supporting planes (151) or supporting arms (152) or supporting rod (153) matched each other.

4. The one-way valve according to claim 3, further comprising a hole (16) in said supporting body (13), which allows gas to pass through.

5. The one-way valve according to claim 3, further comprising a supporting tendon (17), provided on the inner of said supporting body (13).

6. The one-way valve according to claim 3, further comprising a secondary tendon (18), provided on the outer portion of said supporting body (13).

7. The one-way valve according to claim 1, wherein said supporting body (13) is a tube-shaped structure with a circular or elliptical cross section.

8. The one-way valve according to claim 5, further comprising a hole (16) in said supporting body (13), which allows gas to pass through.

9. The one-way valve according to claim 1, further comprising a reinforcing tendon (19), provided on the V-shaped wall of V-shaped funnel construction (11).

10. The one-way valve according to claim 1, wherein said one-way valve is made of flexible medical materials selected from a group of medical silica gel, medical Polyurethane, and medical rubber.
